Europäisches Patentamt

European Patent Office

Office européen des brevets

(11) Numéro de publication: **0 435 984 B1**

(12) **FASCICULE DE BREVET EUROPEEN**

(45) Date de publication de fascicule du brevet: **22.03.95** (51) Int. Cl.6: **C07C 2/66**, C07C 2/86, C07C 13/47

(21) Numéro de dépôt: **90910513.2**

(22) Date de dépôt: **23.07.90**

(86) Numéro de dépôt internationale :
**PCT/CH90/00178**

(87) Numéro de publication internationale :
**WO 91/01959 (21.02.91 91/05)**

(54) **CYCLOALKYLATION SELECTIVE DU NAPHTALENE SUR ZEOLITHES.**

(30) Priorité: **26.07.89 FR 8910322**

(43) Date de publication de la demande:
**10.07.91 Bulletin 91/28**

(45) Mention de la délivrance du brevet:
**22.03.95 Bulletin 95/12**

(84) Etats contractants désignés:
**AT BE CH DE DK ES FR GB IT LI LU NL SE**

(56) Documents cités:
**DE-A- 1 934 426**    **DE-A- 2 208 363**
**DE-C- 638 756**    **US-A- 2 229 018**
**US-A- 2 904 607**    **US-A- 3 641 177**
**US-A- 3 786 106**

**CHEMICAL ABSTRACTS, volume 94, No 3, 19 January 1981, Columbus, Ohio, US; SH.G. SADYKHOV et al.: "Interaction of phenol with cycloolefins in the presence of a zeolite-containing catalyst", see page 403, abstract No 15325z**

(73) Titulaire: **MICHELIN RECHERCHE ET TECHNI-
OUE**
**Euro-résidence**
**Grand-Places 14 A**
**CH-1700 Fribourg (CH)**

(72) Inventeur: **SOLOFO, Jonis**
**571, avenue Comté-de-Nice**
**F-34080 Montpellier (FR)**
Inventeur: **MOREAU, Patrice**
**30, rue des Oliviers**
**F-34980 Saint-Gely-du-Fesc (FR)**
Inventeur: **GENEST, Patrick**
**25, avenue Chancel**
**F-34000 Montpellier (FR)**
Inventeur: **FINIELS, Annie**
**254, rue du Pré-aux-Clercs**
**F-34090 Montpellier (FR)**

(74) Mandataire: **Doussaint, Jean-Marie et al**
**MICHELIN & CIE**
**Service K. Brevets**
**23, Place des Carmes**
**F-63040 Clermont-Ferrand Cedex (FR)**

Rank Xerox (UK) Business Services
(3.10/3.09/3.3.3)

## Description

La présente invention concerne un procédé de cycloalkylation sélective du naphtalène en utilisant des zéolithes comme catalyseur. L'invention concerne en particulier un procédé de préparation de composés aromatiques monocycloalkylés et un procédé de préparation de composés aromatiques dicycloalkylés. Les composés aromatiques mono et dicycloalkylés sont utilisés dans de nombreux domaines et il est important de les préparer sélectivement. Des procédés pour la préparation de composes aromatiques cycloalkylés sont connus dans la littérature.

La production de composés aromatiques contenant des groupes alkyles cycliques en utilisant des halogénures d'aluminium est connue, par exemple la synthèse de mono ou dicyclohexylnaphtalènes avec $AlCl_3$, de telles réactions étant décrites en particulier dans les publications suivantes :
- D. Bodroux, Anneles de Chimie 1929, (10) 11, 535 ;
- Charles C. Price, J. Am. Chem. Soc, 1943, 65 , 439;
- E.S. Pokrovskaya, J. Gen. Chem (URSS), 1939, 9 , 1953 .

Ce procédé conduit à un faible rendement, à une absence de sélectivité, d'autre part il n'est pas économique.

Les brevets ou demandes de brevets DE-C-638 756, DE-A-2 208 363, US-A-2 229 018, et US-A 3 786 106 décrivent l'alkylation de composés aromatiques par des oléfines, par exemple cycliques, en présence de catalyseurs qui sont des argiles, par exemple la montmorillonite, du kaolin, des hydrosilicates, des terres décolorantes. Ces procédés ne sont pas sélectifs.

Les documents US-A 2 904 607, US-A 3 641 177, US-A 4 393 262, DE-A 3 334 084, EP-A-280 055, J. Catal. 1986, 101, p. 273 décrivent l'alkylation de produits aromatiques en utilisant des zéolithes comme catalyseurs, avec des agents d'alkylation linéaires, comme par exemple des oléfines ayant de 2 à 4 atomes de carbone, ou le méthanol. Ces documents ne décrivent pas l'alkylation de produits aromatiques avec des groupes alkyles cycliques.

La demande de brevet DE-A-1 934 426 décrit un procédé continu pour préparer des composés aromatiques alkylés. Ce document cite de nombreux catalyseurs de type zéolithique, par exemple des zéolithes naturelles, comme la gmelinite, la dachiardite, la faujasite, l'heulandite, la mordénite, ou des zéolithes synthétiques comme les zéolithes oméga, L et Y. Ce document cite aussi de nombreux composés aromatiques et une grande variété d'agents d'alkylation. Ce procédé est réalisé en deux étapes. Dans une première étape on réalise l'alkylation avec un agent d'alkylation en présence de zéolithe en suspension dans le milieu liquide. L'alkylation n'est pas sélective et on obtient un mélange de mono et de polyalkylaromatiques. Pour remédier à cette absence de sélectivité, dans une deuxième étape le milieu réactionnel provenant de la première étape est mis au contact d'une zéolithe, sans qu'il y ait de suspension, et sans agent alkylant, de façon à effectuer une transalkylation. Malgré cette deuxième étape, la sélectivité globale à la fin de ces deux étapes reste faible.

L'objet de l'invention est de proposer un procédé de cycloalkylation du naphtalène, avec un agent alkylant, en utilisant au moins une zéolithe comme catalyseur, le procédé se caractérisant de façon surprenante à la fois par un taux de conversion élevé et une sélectivité importante, tout en utilisant un rapport agent alkylant/composé aromatique plus important que ce qui est utilisé en général dans l'art antérieur. Ce procédé est caractérisé par les points suivants :

a) on utilise comme catalyseur au moins une zéolithe de structure faujasite ayant des ouvertures de pores supérieures à 6,7 Å, le rapport silice/alumine de cette zéolithe étant supérieur à 2,5 et son taux résiduel en ion(s) alcalin(s) étant inférieur à 3 % ;

b) la réaction est effectuée en discontinu en phase hétérogène liquide/solide à une température comprise entre 20°C et 250°C, sous une pression au plus égale à 30 bars, la ou les zéolithes étant en suspension dans le milieu réactionnel ;

c) le rapport molaire

$$\frac{\text{agent alkylant}}{\text{naphtalène}}$$

est au moins égal à 1.

Les cycloalkyles fixés par réaction sur le naphtalène sont par exemple le cyclopentyle, le cyclohexyle, ces alkyles comportant éventuellement des substituants. Les réactifs d'alkylation doivent au moins comporter une double liaison, ou un groupe réactif, par exemple un halogène, un groupe OH, de tels réactifs étant par exemple le cyclohexène, le chloro ou le bromocyclohexane, le cyclohexanol.

Les zéolithes utilisées dans le procédé conforme à l'invention peuvent être sous forme protonique ou échangées par des cations, notamment des cations de terres rares (par exemple La, Ce, Pr, Gd, Yb).

Le procéde conforme à l'invention est particulièrement adapté à la cycloalkylation du naphtalène, pour obtenir le dicyclohexyl-2,6 naphtalène, le réactif d'alkylation étant par exemple le chlorocyclohexane ou le bromocyclohexane.

Le dicyclohexyl-2,6 naphtalène est très important dans la chimie des polymères car il permet d'obtenir par oxydation des composés contenant des groupes hydroxyles ou carboxyliques en position 2,6, ces produits servant notamment à la synthèse de polyesters ou de polyamides aromatiques.

L'invention sera aisément comprise à l'aide des exemples non limitatifs qui suivent.

Dans ces exemples, on utilise des zéolithes Y de structure faujasite, des zéolithes de structure mordénite, et de structure Omega, toutes ces zéolithes étant sous leur forme protonique. Ces zéolithes sont les suivantes :

- une zéolithe HY, désignée ci-après "HY1", commercialisée par Union Carbide, sous forme $NH_4Y$, sous le nom de Linde SK 41 ;
- une zéolithe HY, désignée ci-après "HY2", commercialisée par Chemische Fabrik Uetikon sous forme HY sous le nom de Z6-03-02 ;
- une zéolithe US-HY, qui est une zéolithe ultra-stable obtenue en traitent la zéolithe HY2 selon le procédé décrit par J. Scherzer et J.L. Bass dans J. Catal, 1973, 28, 101 ; cette zéolithe US-HY ayant un taux résiduel en ion(s) alcalin(s) inférieur à 1 % ;
- une zéolithe de structure mordénite, désignée ci-après "MOR 1", commercialisée par la Société Norton sous le nom de Zéolon 100 H ;
- une zéolithe de structure mordénite, désignée ci-après "MOR 2", obtenue à partir de la zéolithe MOR 1 par traitement avec de l'acide chlorhydrique 1N pendant 3 heures à 70°C ;
- une zéolithe de structure Omega, ci-après désignée "OMEGA" préparée conformément à la demande de brevet français n° 85-07772, et activée suivant le procédé décrit dans J. Catal. 111, 94-105 (1988).

Avant chaque réaction, les zéolithes subissent un traitement thermique de calcination sous un courant d'air de 200 cm$^3$/minute. La calcination est opérée suivant un des deux procédés suivants :

- calcination pendant 6 heures à 300°C (zéolithes US-HY, HY1 et HY2) ;
- calcination pendant 6 heures à 500°C (pour les autres zéolithes).

Cette calcination est effectuée dans un four permettant une montée linéaire de la température de 50°C/heure, à partir de la température ambiante, le temps de montée en température s'ajoutant aux temps de calcination précédemment indiqués.

Les caractéristiques des zéolithes calcinées, prêtes à l'emploi sont données dans le tableau I suivant :

TABLEAU I

| Zéolithe | Caractéristiques (Poudre) | | |
|---|---|---|---|
| | % Na2O (% en poids) | %Al2O3 (% en poids) | % SiO2 (% en poids) |
| HY1 | 2,5 | 22,3 | 64,9 |
| HY2 | 2,5 | 23,5 | 72,5 |
| US-HY | < 0,2 | 25 | 75 |
| MOR1 | 0,4 | 9 | 72 |
| MOR2 | 0,01 | 7,1 | 92,8 |
| OMEGA | 0,3 | 22,1 | 66,3 |

TABLEAU I (suite)

| Zéolithe | Caractéristiques (Poudre) | | |
|---|---|---|---|
| | $\frac{SiO2}{Al2O3}$ (massique) | Surface spécifique $(m^2/g)$ | Ouverture des pores (Å) |
| HY1 | 2,9 | 948 | 7,4 |
| HY2 | 3,0 | 700 | 7,4 |
| US-HY | 3,0 | 675 | 7,4 |
| MOR1 | 8,0 | 550 | 6,7 x 7,0 |
| MOR2 | 13,0 | 510 | 6,7 x 7,0 |
| OMEGA | 3,0 | 140 | 8 |

Sauf indication contraire, tous les essais d'alkylation décrits dans les exemples qui suivent sont réalisés dans un autoclave de 100 ml de la Société BURTON-CORBLIN, muni d'un agitateur rotatif interne à entraînement magnétique, d'une vanne de prélèvements, d'un manomètre et d'un appareil de mesure de vitesse de rotation. L'autoclave est chauffé par un four dont la régulation de température est assurée par un régulateur de la société SOTELEM.

Les analyses sont effectuées avec un chromatographe en phase gazeuse DELSI série 330 équipé d'une colonne capillaire OVI de 25 m, muni d'un détecteur à ionisation de flamme. Il est couplé à un intégrateur DELSI ENICA 21. Pour chaque essai, le milieu réactionnel est analysé au moins deux fois. Le nitrobenzène est utilisé comme étalon interne, et il est rajouté dans les prélèvements servant à l'analyse.

Les conditions d'analyse chromatographique sont les suivantes :
- température de l'injecteur : 330°C ;
- température du détecteur : 320°C ;
- programmation de la température du four : adaptée aux composés, par exemple, pour le naphtalène : chauffage de 100°C à 280°C, la vitesse de chauffage étant de 15°C/min ;
- pression de l'hydrogène servant de gaz vecteur : 0,65 bar.

La détermination de la structure des produits de la réaction est effectuée par un ensemble constitué par un chromatographe en phase gazeuse couplé avec un spectromètre de masse. Le chromatographe est équipé d'une colonne capillaire de type OVI de 25 m.

Les conditions d'analyse avec cet ensemble sont les suivantes :
- température de l'injecteur : 250°C ;
- température du détecteur : 250°C ;
- programmation de la température du four : adaptée aux composés, par exemple, pour le naphtalène : chauffage de 100°C à 280°C, avec une montée en température de 10°C/min ;
- pression de l'hélium utilisé comme gaz vecteur : 0,5 bar.

Exemple 1

Le but de cet exemple est de comparer les résultats obtenus avec les zéolithes précédemment décrites dans le cas de la cyclohexylation du naphtalène, en utilisant le bromocyclohexane comme agent alkylant et le cyclohexane comme solvant.

Pour chaque zéolithe la réaction est effectuée de la façon suivante :
On introduit dans l'autoclave 0,64 g de naphtalène (5 mmoles), 50 cm$^3$ de cyclohexane, 1 g de zéolithe en poudre calcinée et 1,62 g de bromocyclohexane (10 mmoles). On ferme l'autoclave et on agite à 680 tours/min pour obtenir une suspension du catalyseur dans le milieu réactionnel, tout en chauffant de telle sorte que la température du milieu réactionnel passe de 25°C à 200°C en 10 min. La pression autogène est alors de 15 bars à l'intérieur de l'autoclave. On effectue alors des prélèvements du mélange réactionnel pour analyse, dans le cas des essais 1 à 4, et on refroidit l'autoclave. Pour ce qui est des essais 5 et 6, les prélèvements sont effectués 70 minutes après le début de l'agitation et du chauffage, la température de l'autoclave étant maintenue à 200°C pendant 60 minutes avant ces prélèvements, la pression restant égale à 15 bars. Dans tous les cas, après refroidissement, on filtre le milieu réactionnel pour séparer la poudre de zéolithe qu'on lave avec 50 ml d'éther. On réunit cet éther de lavage au filtrat pour obtenir une phase organique sur laquelle on effectue des prélèvements pour faire une analyse qui coïncide avec l'analyse du dernier prélèvement effectué.

Les résultats des essais sont donnés dans le tableau II. Dans ce tableau II, comme dans les autres tableaux qui suivent, la conversion totale est exprimée en % molaire. De façon générale, on entend par conversion totale (taux de conversion) le rapport molaire :

$$\frac{\text{Nombre de moles de naphtalènes cycloalkylés}}{\text{Nombre de moles de naphtalène de départ}}$$

la distribution des produits est exprimée en % molaire, ainsi que le rapport des produits.

Tableau II

| N° d'essai | 1 | 2 | 3 |
|---|---|---|---|
| **Conditions de réaction** | | | |
| Température finale °C | 200 | 200 | 200 |
| Temps de réaction (min) | 10 | 10 | 10 |
| Rapport molaire : | | | |
| $\dfrac{\text{agent alkylant}}{\text{naphtalène}}$ | 2 | 2 | 2 |
| catalyseur | HY1 | HY2 | US-HY |
| **Conversion totale** | 83 | 96 | 96 |
| **Distribution des produits** | | | |
| Monoalkylés | 71 | 65 | 31 |
| Dialkylés | 16 | 31 | 67 |
| Autres | 13 | 4 | 2 |
| **Rapport des produits** | | | |
| $\dfrac{\text{Monocyclohexyl-2 naphtalène}}{\text{Monocyclohexyl naphtalenes}}$ | 46 | 18 | 6 |
| $\dfrac{\text{Dicyclohexyl-2,6 naphtalène}}{\text{Dicyclohexylnaphtalènes}}$ | 11 | 33 | 43 |
| $\dfrac{\text{Dicyclohexyl-2,6 + dicyclohexyl-2,7 naphtalènes}}{\text{Dicyclohexylnaphtalènes}}$ | 25 | 65 | 82 |

TABLEAU II (suite)

| N° d'essai | 4 | 5 | 6 |
|---|---|---|---|
| **Conditions de réaction** | | | |
| Température finale °C | 200 | 200 | 200 |
| Temps de réaction (min) | 10 | 70 | 70 |
| Rapport molaire : | | | |
| agent alkylant / naphtalène | 2 | 2 | 2 |
| catalyseur | MOR1 | MOR2 | OMEGA |
| **Conversion totale** | < 1 | 6 | 0 |
| **Distribution des produits** | | | |
| Monoalkylés | – | 56 | – |
| Dialkylés | – | 44 | – |
| Autres | – | – | – |
| **Rapport des produits** | | | |
| Monocyclohexyl-2 naphtalène / Monocyclohexyl naphtalènes | – | 46 | – |
| Dicyclohexyl-2,6 naphtalène / Dicyclohexylnaphtalènes | – | 16 | – |
| Dicyclohexyl-2,6 + dicyclohexyl-2,7 naphtalènes / Dicyclohexylnaphtalènes | – | 27 | – |

Les essais 1 à 3 avec les zéolithes HY1, HY2, US-HY sont conformes à l'invention, les essais 4 à 6 avec les zéolithes MOR1, MOR2, OMEGA ne sont pas conformes à l'invention.

On constate sur le tableau II que le procédé conforme à l'invention permet, de façon inattendue, d'obtenir l'ensemble des avantages suivants :
- le taux de conversion totale est très élevé (de 83 à 96 %) alors que les zéolithes MOR1, MOR2 et OMEGA conduisent à des taux de conversion très faibles ou nuls (de 0 à 6 %);
- la sélectivité est grande, en effet les zéolithes HY1 et HY2 conduisent à une forte proportion de monocyclohexyl naphtalènes (71 % et 65 %) alors que la zéolithe US-HY conduit à une forte proportion de dicyclohexylnaphtalènes (67 %).

La zéolithe US-HY est particulièrement intéressante sur le plan de la sélectivité, car non seulement elle conduit à une dicyclohexylation prédominante, mais en outre à une sélectivité en isomères 2,6 + 2,7 importante (82 %) par rapport à l'ensemble des dicyclohexylnaphtalènes. Les catalyseurs HY2 et US-HY ont en outre l'avantage de conduire à des pourcentages très faibles de naphtalènes tri et tétrasubstitués.

- Cette conversion et cette sélectivité importantes sont obtenues avec un rapport

$$\frac{\underline{agent\ alkylant}}{naphtalène}$$

élevé, ce qui est très avantageux sur le plan économique.

Exemple 2

Cet exemple est réalisé de façon analogue à l'exemple 1 avec les zéolithes HY1 et US-HY, avec la différence que l'on utilise un réacteur ouvert, à la pression atmosphérique, au lieu de l'autoclave, que pour chaque essai on utilise 20 mmoles de bromocyclohexane et qu'on effectue la réaction pendant 6 heures à 80°C. On ajoute la poudre de zéolithe au mélange naphtalène, cylohexane chauffé prélablement à 80°C avant de faire l'addition du bromocyclohexane.

Les résultats des essais sont donnés dans le tableau III.

### Tableau III

| N° d'essai | 7 | 8 |
|---|---|---|
| Conditions de réaction | | |
| Température °C | 80 | 80 |
| Temps de réaction (min) | 360 | 360 |
| Agent d'alkylation | Bromocyclohexane | |
| Rapport molaire : | | |
| $\dfrac{agent\ alkylant}{naphtalène}$ | 4 | 4 |
| catalyseur | HY1 | US-HY |
| Conversion totale | 57 | 84 |
| Distribution des produits | | |
| Monocyclohexylnaphtalènes | 96 | 90 |
| Dicyclohexylnaphtalènes | 4 | 10 |
| Rapport des produits | | |
| $\dfrac{Cyclohexyl-2\ naphtalène}{Monocyclohexylnaphtalènes}$ | 60 | 38 |

L'examen du tableau III montre ici encore que le procédé conforme à l'invention permet un taux de conversion total élevé avec les deux zéolithes, nais la réaction étant ici orientée vers la monocyclohexylation par suite de la faible température La zéolithe US-HY permet un taux de conversion totale plus élevé que celui obtenu avec la zéolithe HY1 (84 % pour l'US-HY, 57 % pour la HY1), la sélectivité en monocyclohexylnaphtalènes étant du même ordre pour les deux zéolithes.

Exemple 3

Cet exemple est réalisé en alkylant du naphtalène en faisant varier l'agent d'alkylation, avec les zéolithes US-HY, HY1, HY2. Les agents d'alkylation utilisés sont les suivants : bromocyclohexane (BCH), chlorocyclohexane (CCH), bromocyclopentane (BCP), cyclohexanol (CHL), chlorure de benzyle (CBZ).
Les conditions d'essais sont les suivantes :
- quantités utilisées 0,64 g de naphtalène (5 mmoles), 50 cm$^3$ de cyclohexane, 1,0 g de zéolithe, 10 mmoles d'agent d'alkylation,
- agitation du réacteur : 680 tours/min,
- mise en route du réacteur à la température ambiante, analyse des produits de réaction effectuée lorsque la température du milieu réactionnel atteint 200°C, c'est-à-dire 10 min après la mise en route du réacteur, et donc après le début du chauffage, la pression autogène dans le réacteur étant alors de 15 bars.

Les résultats sont donnés dans le tableau IV.

<div align="center"><u>TABLEAU IV</u></div>

| N° d'essai | 9 | 10 | 11 |
|---|---|---|---|
| <u>Conditions de réaction</u> | | | |
| Température finale °C | 200 | 200 | 200 |
| Temps de réaction (min) | 10 | 10 | 10 |
| Agent d'alkylation | BCH | BCH | BCH |
| Rapport molaire : | | | |
| <u>agent alkylant</u><br>naphtalène | 2 | 2 | 2 |
| catalyseur | US-HY | HY2 | HY1 |
| <u>Conversion totale</u> | 96 | 96 | 83 |
| <u>Distribution des produits</u> | | | |
| Monoalkylés | 31 | 65 | 71 |
| Dialkylés | 67 | 31 | 16 |
| Trialkylés | - | - | - |
| Autres | 2 | 4 | 13 |
| <u>Rapport des produits</u> | | | |
| <u>Monoalkyl-2 naphtalène</u><br>Monoalkyl naphtalènes | 6 | 18 | 46 |
| <u>Dialkyl-2,6 naphtalène</u><br>Dialkyl naphtalènes | 43 | 33 | 11 |
| Dialkyl-2,6 +<br><u>Dialkyl-2,7 naphtalènes</u><br>Dialkyl naphtalènes | 82 | 65 | 25 |

TABLEAU IV (suite)

| N° d'essai | 12 | 13 | 14 |
|---|---|---|---|
| Conditions de réaction | | | |
| Température finale °C | 200 | 200 | 200 |
| Temps de réaction (min) | 10 | 10 | 10 |
| Agent d'alkylation | CCH | CCH | CCH |
| Rapport molaire : | | | |
| agent alkylant / naphtalène | 2 | 2 | 2 |
| catalyseur | US-HY | HY2 | HY1 |
| Conversion totale | 97 | 89 | 78 |
| Distribution des produits | | | |
| Monoalkylés | 62 | 76 | 72 |
| Dialkylés | 38 | 24 | 11 |
| Trialkylés | - | - | - |
| Autres | - | - | 17 |
| Rapport des produits | | | |
| Monoalkyl-2 naphtalène / Monoalkyl naphtalènes | 22 | 41 | 46 |
| Dialkyl-2,6 naphtalène / Dialkyl naphtalènes | 38 | 21 | 21 |
| Dialkyl-2,6 + Dialkyl-2,7 naphtalènes / Dialkyl naphtalènes | 59 | 37 | 35 |

11

EP 0 435 984 B1

TABLEAU IV (suite)

| N° d'essai | 15 | 16 | 17 |
|---|---|---|---|
| Conditions de réaction | | | |
| Température finale °C | 200 | 200 | 200 |
| Temps de réaction (min) | 10 | 10 | 10 |
| Agent d'alkylation | BCP | BCP | CHL |
| Rapport molaire : | | | |
| agent alkylant / naphtalène | 2 | 2 | 2 |
| catalyseur | US-HY | HY2 | HY1 |
| Conversion totale | 97 | 91 | 28 |
| Distribution des produits | | | |
| Monoalkylés | 25 | 50 | 75 |
| Dialkylés | 58 | 47 | 3 |
| Trialkylés | 3 | 2 | - |
| Autres | 14 | 1 | 22 |
| Rapports des produits | | | |
| Monoalkyl-2 naphtalène / Monoalkyl naphtalènes | 0 | 44 | 47 |
| Dialkyl-2,6 naphtalène / Dialkyl naphtalènes | 39 | 27 | 27 |
| Dialkyl-2,6 + Dialkyl-2,7 naphtalènes / Dialkyl naphtalènes | 84 | 47 | 41 |

12

TABLEAU IV (suite)

| N° d'essai | 18 |
|---|---|
| **Conditions de réaction** | |
| Température finale °C | 200 |
| Temps de réaction (min) | 10 |
| Agent d'alkylation | CBZ |
| Rapport molaire : | |
| $\dfrac{\text{agent alkylant}}{\text{naphtalène}}$ | 2 |
| catalyseur | US-HY |
| **Conversion totale** | 12 |
| **Distribution des produits** | |
| Monoalkylés | 100 |
| Dialkylés | – |
| Trialkylés | – |
| Autres | – |
| **Rapport des produits** | |
| $\dfrac{\text{Monoalkyl-2 naphtalène}}{\text{Monoalkyl naphtalènes}}$ | 80 |
| $\dfrac{\text{Dialkyl-2,6 naphtalène}}{\text{Dialkyl naphtalènes}}$ | -- |
| $\dfrac{\text{Dialkyl-2,6 + dialkyl-2,7 naphtalènes}}{\text{Dialkyl naphtalènes}}$ | – |

Tous les essais sont conformes à l'invention sauf lorsque l'agent d'alkylation est le chlorure de benzyle qui n'est pas un agent de cycloalkylation (CBZ essai n° 18).

Pour tous les essais conformes à l'invention, sauf lorsque l'agent d'alkylation est le cyclohexanol (CHL), le taux de conversion totale est très élevé, la zéolithe US-HY conduisant dans chaque cas à une meilleure sélectivité en produits dicycloalkylés (67 %, 38 %, 58 %) que les autres zéolithes, qui sont cependant sélectives pour l'obtention de produits mono et/ou dicycloalkylés.

Dans le cas du cyclohexanol, la conversion totale est moins élevée, tout en étant substantielle.

La réaction avec le chlorure de benzyle (CBZ), non conforme à l'invention, conduit par contre à un taux de conversion totale très faible.

Dans les exemples précédents conformes à l'invention, les divers produits obtenus peuvent être purifiés et séparés par des méthodes conventionnelles comme par exemple des distillations sous vide ou des cristallisations.

De préférence, la température d'alkylation est au moins égale à 50°C et au plus égale à 220°C. De préférence, la température est au moins égale à 50°C et au plus égale à 140°C si on désire essentiellement ou en totalité une monoalkylation et supérieure à 140°C et au plus égale à 220°C, si on veut essentiellement ou en totalité une dialkylation.

De préférence, dans le procédé conforme à l'invention, le taux de conversion est supérieur à 50 % et il permet d'obtenir plus de 50 % soit de monocycloalkylnaphtalènes, soit de dicycloalkylnaphtalènes, par rapport à l'ensemble des cycloalkylnaphtalènes obtenus, ces pourcentages étant molaires. Avantageusement, le taux de conversion est supérieur à 80 % et le procédé permet d'obtenir plus de 60 % soit de monocycloalkylnaphtalènes, soit de dicycloalkylnaphtalènes, en rapports molaires, par rapport à l'ensemble des cycloalkylnaphtalènes obtenus.

**Revendications**

1.  Procédé pour cycloalkyler le naphtalène avec au moins un groupe cyclique, caractérisé par les points suivants :

    a) on utilise comme catalyseur au moins une zéolithe de structure faujasite ayant des ouvertures de pores supérieures à 6,7 Å, le rapport silice/alumine de cette zéolithe étant supérieur à 2,5 et son taux résiduel en ion(s) alcalin(s) étant inférieur à 3 % ;

    b) la réaction est effectuée en discontinu en phase hétérogène liquide/solide à une température comprise entre 20°C et 250°C, sous une pression au plus égale à 30 bars, la/ou les zéolithes étant en suspension dans le milieu réactionnel ;

    c) le rapport molaire

$$\frac{\text{agent alkylant}}{\text{naphtalène}}$$

    est au moins égal à 1.

2.  Procédé conforme à la revendication 1, caractérisé en ce que la zéolithe a un taux résiduel en ion(s) alcalin(s) inférieur à 1 %.

3.  Procédé selon l'une quelconque des revendications 1 ou 2, caractérisé en ce que la température est au moins égale à 50°C et au plus égale à 220°C.

4.  Procédé selon la revendication 3, caractérisé en ce que l'alkylation est essentiellement ou en totalité une monoalkylation.

5.  Procédé selon la revendication 4, caractérisé en ce que la température est au moins égale à 50°C et au plus égale à 140°C.

6.  Procédé selon la revendication 3, pour préparer un dicycloalkyl naphtalène.

7.  Procédé selon la revendication 6, caractérisé en ce que la température est supérieure à 140°C et au plus égale à 220°C.

8.  Procédé selon l'une quelconque des revendications 1 à 7, caractérisé en ce que le cycloalkyle est le cyclohexyle.

9.  Procédé selon la revendication 8, pour préparer du dicyclohexyl-2,6 naphtalène.

**Claims**

1. A method for cycloalkylating naphthalene with at least one cyclic group, characterised by the following points:

   (a) there is used as catalyst at least one zeolite of faujasite structure having pore openings of more than 6.7 Å, the silica/alumina ratio of this zeolite being greater than 2.5, and its residual concentration in alkaline ion(s) being less than 3%;

   (b) the reaction is carried out discontinuously in heterogenous liquid/solid phase at a temperature of between 20°C and 250°C at a pressure of at most 30 bar, the zeolite or zeolites being suspended in the reaction medium;

   (c) the molar ratio of alkylating agent to naphthalene is at least equal to 1.

2. A method according to Claim 1, characterised in that the zeolite has a residual content of alkaline ion(s) of less than 1%.

3. A method according to any one of Claims 1 or 2, characterised in that the temperature is at least equal to 50°C and at most equal to 220°C.

4. A method according to Claim 3, characterised in that the alkylation is essentially or entirely a monoalkylation.

5. A method according to Claim 4, characterised in that the temperature is at least equal to 50°C and at most equal to 140°C.

6. A method according to Claim 3 for preparing a dicycloalkyl naphthalene.

7. A method according to Claim 6, characterised in that the temperature is greater than 140°C and at most equal to 220°C.

8. A method according to any one of Claims 1 to 7, characterised in that the cycloalkyl is cyclohexyl.

9. A method according to Claim 8 for preparing 2,6-dicyclohexyl naphthalene.

**Patentansprüche**

1. Verfahren zur Cycloalkylierung von Naphthalin unter Einführung mindestens einer cyclischen Gruppe, gekennzeichnet durch folgende Merkmale:

   (a) als Katalysator wird mindestens ein Zeolith mit Faujasit-Struktur verwendet, der eine Porengröße von mehr als 6,7 Å, ein Siliciumdioxid/Aluminiumoxid-Verhältnis von mehr als 2,5 und einen Restgehalt an einem oder mehreren Alkaliionen unter 3 % aufweist;

   (b) die Reaktion wird diskontinuierlich in einer heterogenen Fest-Flüssig-Phase bei einer Temperatur zwischen 20 und 250 °C unter einem Druck von höchstens 30 bar durchgeführt, wobei sich der Zeolith bzw. die Zeolithe in Suspension im Reaktionsmedium befinden;

   (c) das Molverhältnis Alkylierungsmittel/Naphthalin ist mindestens gleich 1.

2. Verfahren nach Anspruch 1, dadurch gekennzeichnet, daß der Zeolith einen Restgehalt an einem oder mehreren Alkaliionen unter 1 % aufweist.

3. Verfahren nach Anspruch 1 oder 2, dadurch gekennzeichnet, daß die Temperatur mindestens gleich 50 °C und höchstens gleich 220 °C ist.

4. Verfahren nach Anspruch 3, dadurch gekennzeichnet, daß die Alkylierung im wesentlichen oder ausschließlich eine Monoalkylierung ist.

5. Verfahren nach Anspruch 4, dadurch gekennzeichnet, daß die Temperatur mindestens gleich 50 °C und höchstens gleich 140 °C ist.

6. Verfahren nach Anspruch 3 zur Herstellung von ein Dicycloalkylnaphthalin.

7. Verfahren nach Anspruch 6, dadurch gekennzeichnet, daß die Temperatur mehr als 140 °C und höchstens 220 °C beträgt.

8. Verfahren nach einem der Ansprüche 1 bis 7, dadurch gekennzeichnet, daß die Cycloalkylgruppe Cyclohexylgruppe ist.

9. Verfahren nach Anspruch 8 zur Herstellung von 2,6-Dicyclohexylnaphthalin.